(19) Europäisches Patentamt
European Patent Office
Office européen des brevets

(11) Veröffentlichungsnummer: **0 002 498**
**B1**

(12) # EUROPÄISCHE PATENTSCHRIFT

(45) Veröffentlichungstag der Patentschrift: **09.09.81**

(21) Anmeldenummer: **78101591.2**

(22) Anmeldetag: **07.12.78**

(51) Int. Cl.³: **C 07 C 31/20,**
C 07 C 29/04, C 11 B 9/00

(54) **Verfahren zur Herstellung von Hydroxycitronellol.**

(30) Priorität: **15.12.77 DE 2755945**

(43) Veröffentlichungstag der Anmeldung:
**27.06.79 Patentblatt 79/13**

(45) Bekanntmachung des Hinweises auf die Patenterteilung:
**09.09.81 Patentblatt 81/36**

(84) Benannte Vertragsstaaten:
**CH DE FR GB NL**

(73) Patentinhaber: **BASF Aktiengesellschaft**
**Carl-Bosch-Strasse 38**
**D-6700 Ludwigshafen (DE)**

(72) Erfinder: **Hoffmann, Werner, Dr.**
**Ringstrasse 11 c**
**D-6701 Neuhofen (DE)**

(56) Entgegenhaltungen:
**US - A - 3 257 469**
**US - A - 3 285 977**

**CHEMICAL ABSTRACTS, Band 81, Nr. 7. 19. August 1974 Columbus, Ohio, USA MATSUBARA YOSHIHARU et al; "Hydration of terpenes, II. Hydration of various terpene hydrocarbons and alcohols with a cation exchange resin" Seite 398, linke Spalte, Zusammenfassung Nr. 37653p**

**CHEMICAL ABSTRACTS, Band 87, Nr. 17, 24. Oktober 1977, Columbus, Ohio, USA ICHIKAWA YATARO et al., "Hydroxycitronellol from citronellol", Seite 775 linke Spalte, Zusammenfassung Nr. 136047z**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 002 498 B1

Courier Press, Leamington Spa, England.

Verfahren zur Herstellung von Hydroxycitronellol

Die vorliegende Erfindung betrifft ein verbessertes Verfahren zur Herstellung von Hydroxycitronellol (I; 3,7-Dimethyl-octan-1,7-diol) durch Wasseranlagerung an Citronellol (II; 3,7-Dimethyl-oct-6-en-1-ol)

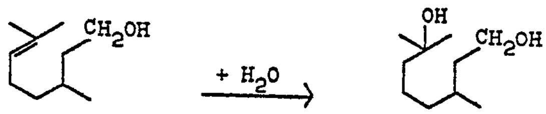


II I

in Gegenwart eines Kationenaustauschers.

Aus "Yuki Gosei Kagaku Kyokai" Shi 31 (1973) 11, 924 — 27 ist es bekannt, das als Duftstoff und insbesondere als Zwischenprodukt für die Herstellung des als Duftstoff sehr begehrten Hydroxycitronellals benötigte Hydroxycitronellol I herzustellen, indem man an Citronellol eine niedere Carbonsäure wie Essigsäure in Gegenwart eines Kationenaustauschers anlagert und das Reaktionsprodukt anschließend verseift. Dieses Verfahren hat jedoch den Nachteil, daß einerseits nur geringe Umsätze erzielt werden und andereseits nicht Hydroxycitronellol selbst, sondern dessen Carbonsäureester gebildet werden, die nach ihrer Isolierung erst verseift werden müssen, eine Verfahrensweise, die technisch in hohem Maße unwirtschaftlich ist.

"Ferner ist es aus den US-Patentschriften 3 257 469 und 3 285 977 bekannt, Olefine in Gegenwart von sauren Ionenaustauschern und Lösungsvermittlern wie Isopropanol durch Hydratisierung in Alkohole zu überführen. Da Olefine jedoch keine weiteren funktionellen Gruppen tragen sind unerwünschte Nebenreaktionen wie Veretherungen primärer Hydroxylgruppen mit dem alkoholischen Lösungsvermittler in diesen Fällen nicht zu befürchten. Überdies sind die Ausbeuten des nach diesen Verfahren hergestellten Alkohols so unbefriedigend, daß man aus den Lehren dieser US-Patentschriften keine allgemeinen Prinzipien für technisch brauchbare Verfahren ableiten kann."

Der Erfindung lag daher die Aufgabe zugrund, die Herstellung von Hydroxycitronellol unter Vermeidung der geschilderten Nachteile auf möglichst einfache und daher billige Weise herzustellen.

Es wurde nun überraschenderweise gefunden, daß man Hydroxycitronellol (I) durch Anlagerung vcn Wasser an Citronellol (II) auf wirtschaftliche und technisch einfache Weise erhält, wenn man eine homogen Lösung, enthaltend pro Kilogramm II 0,1 bis 10 Liter, vorzugsweise 1 bis 5 Liter Wasser sowie ein $\alpha$-methylverzweigtes primäres, sekundäres oder tertiäres $C_3$—$C_5$-Alkanol bei 40 bis 120°C mit einem stark sauren Kationenaustauscher behandelt und das Reaktionsgemisch danach wie üblich destillativ auf das gewünschte Verfahrensprodukt aufarbeitet.

Bei dem erfindungsgemäßen Verfahren kann also überraschenderweise auf die Mitverwendung der relativ teuren niederen Carbonsäuren sowie auf die spätere aufwendige Verseifung der gebildeten Carbonsäureester verzichtet werden.

Die Ausgangsverbindung II ist als natürliches oder synthetisches Citronellol handelsüblich.

Als definitionsgemäße Alkanole eignen sich vor allem Propan-2-ol, 2-Methyl-propanol (Isobutanol), 2-Methyl-propan-2-ol (tert. Butanol) und Butanol-(2).

Die Menge dieser Alkanole wird zweckmäßigerweise so bemessen, daß sich das Gemisch aus II und Wasser bei Raumtemperatur gerade homogen in ihnen löst. Je nach Art des Alkanols und der Menge des verwendeten Wassers sind hierzu etwa 5 bis 20 Liter Alkanol pro Kilogramm II erforderlich. Größere Menge, etwa bis zur doppelten Menge, schaden nicht, bieten aber im allgemeinen auch keinen Vorteil.

Die Menge des Wassers hat nach bekannten Gesetzmäßigkeiten breiten Einfluß auf die Reaktionsgeschwindigkeit. Innerhalb des oben angegebenen Bereiches erzielt man besonders gute Ergebnisse bei Verwendung von 1 bis 5 Liter Wasser pro Kilogramm II. Größere Mengen als 10 Liter Wasser pro Kilogramm II sind aus wirtschaftlichen Gründen nicht sinnvoll, da sie ein zu großes Flüssigkeitsvolumen bedingen.

Als stark saure Kationenaustauscher verstehen wir erfindungsgemäß Kunstharz-Ionenaustauscher, d.h. hochpolymere räumliche Netzwerke aus Kohlenstoffketten (Matrix) in Gelstruktur, die als ladungstragende Gruppen (Festionen) —$SO_3^{\ominus}$-Gruppen oder —$SO_3^{\ominus}$- und —$O^{\ominus}$-Gruppen enthalten. Es handelt sich hierbei im wesentlichen um handelsübliche Kationenaustauscher auf der Basis von Polystyrolsulfonsäureharzen oder Phenolsulfonsäureharzen, die z.B. unter folgenden Handelsnamen bekannt sind: Lewatit S100, Lewatit S 115, Lewatit SP 1080, Lewatit SC 102, Lewatit SPC 118, Amberlite IR 120, Amberlite IR 200, Aberlyst 15, Dowex 50, Permutit RS, Wofatit KPS 200, Duolite C—3, Duolite C—10, Duolite C—25, Wofatit F, Wofatit D, Wofatit P, Zeoxex (Keokarb H), Nalcite HCR, Nalcite HGR, Nalcite HDR, Permutit Q und Permutit RS.

Besonders günstige Ergebnisse bezüglich Umsatz und Reaktionszeit wurden mit solchen Kationenaustauscher erzielt, bei denen die Zahl der aktiven Zentren pro Oberflächeneinheit besonders groß ist. Hierzu zählen z.B. die besonders feinteiligen Kationenaustauscher, wie Lewatit SPC 108, Lewatit SP 1080 und Lewatit SPC 118 sowie die sogenannten großporigen Ionenaustauscher wie Amberlyst 15.

Der Kationenaustauscher wird in der handelsüblichen wasserhaltigen Form eingesetzt, jedoch vor der Umsetzung mit etwa 5- bis 10-fachem Volumen des entsprechenden Alkohol-Wasser-Gemischs gewaschen.

Bezüglich näherer Einzelheiten über Herstellung, Eigenschaften und Verwendung der sauren Kationenaustauscher verweisen wir auf Ullmanns Encyclopädie der technischen Chemie, 3. Auflage, Band 8, 1957, Seite 787 ff, insbesondere Seite 806—811 und 814—822.

Für die diskontinuierliche Arbeitsweise verwendet man diese Harze zweckmäßigerweise in Mengen von 1 bis 5 l pro Kilogramm II. Nimmt man die Umsetzung kontinuierlich vor, indem man etwa die wäßrig-alkoholische Lösung von II durch Austauschersäulen leitet, so richtet man es zweckmäßigerweise so ein, daß 1 kg II pro Stunde über 5 bis 10 l des Harzes geleitet werden. Vor erstmaligem Gebrauch wäscht man die Austauscherharze zweckmäßigerweise mit einer wäßrigen Lösung des Alkanols, deren Zusammensetzung etwa dem Wasser/Alkanol-Verhältnis in den II-Lösungen entspricht.

Die bevorzugten Reaktionstemperaturen liegen zwischen 50 und 90°C. Da es sich um eine Gleichgewichtsreaktion handelt, erzielt man keine vollständigen Umsätze, sondern, je nach Temperatur und Wassermenge maximal Umsätze zwischen 40 und 70%. Die wirtschaftlich optimalen Umsätze liegen bei etwa 50 bis 65% der Gleichgewichtskonzentration, denn bei höheren Umsätzen verlangsamt sich die Reaktion in Relation zur Umsatzerhöhung zu sehr, Diese Umsätze erfordern je nach der Menge der Kationenaustauscher Reaktionszeiten von etwa 1 bis 20 Stunden. Der Grad der Umsetzung kann gaschromatographisch verfolgt werden.

Nach Erreichen des gewünschten Umsatzes trennt man die Lösung vom Kationenaustauscher ab und arbeitet sie wie üblich destillativ auf. Die nicht umgesetzte Ausgangsverbindung, das Alkanol und das Wasser werden zweckmäßigerweise in die Reaktionsstufe zurückgeführt. Der Kationenaustausch behält über lange Betriebszeiten seine Aktivität unverändert bei und braucht deshalb nur gelegentlich wie üblich regeneriert zu werden.

Das nach dem erfindungsgemäßen Verfahren erhältlich Hydroxycitronellol ist als Duftstoff verwendbar und hat als Zwischenprodukt für die Herstellung des begehrten Hydroxycitronellals Bedeutung.

Hydroxycitronellal wird nach dem erfindungsgemäßen Verfahren auf technisch einfache und billige Weise erhalten.

Beispiel 1

Eine Lösung aus 50 g Citronellol, 200 ml Wasser und 300 ml Isopropanol wurde 17 Stunden lang bei 80°C mit 100 ml des stark sauren Kationenaustauschers Lewatit S100 (Teilchengröße: 0,3—1 mm), der zuvor mit dem 5-fachen Volumen eines Isopropanol-Wasser-Gemisches (Mischungsverhältnis 3:2) ausgewaschen worden war, gerührt. Nach 17 Stunden waren ca. 61% Citronellol umgesetzt und die Gleichgewichtseinstellung erreicht.

Die nach Abtrennung des Austauscherharzes übliche destillative Aufarbeitung der Lösung ergab 19 g unumgesetztes Citronellol und 32 g Hydroxycitronellol, was einer Ausbeute von 94%, bezogen auf umgesetztes Citronellol, entspricht. Kp bei 0,03 mbar = 99 — 101°C; $n_D^{25}$ = 1,4579.

Beispiel 2 bis 8

Eine Lösung aus jeweils 50 g Citronellol, 200 ml Wasser und 300 ml Isopropanol wurde in der aus der Tabelle ersichtlichen Reaktionszeit bei 80°C mit dem aus der Tabelle ersichtlichen Kationenaustauscher, der analog Beispiel 1 vorbehandelt war, gerührt und das vom Austauscherharz abgetrennte Reaktionsgemisch destillativ aufgearbeitet. Die erzielten Ergebnisse wurden in der folgenden Tabelle dargestellt:

| Kationenaustauscher | Reaktionszeit [h] | Umsatz | Ausbeute, bezogen auf umgesetztes II |
|---|---|---|---|
| Lewatit SP 120 | 12 | 62% | 92—94% |
| Lewatit SPC 108 | 5 | 64% | " |
| Amberlyst 15 | 8 | 61% | " |
| Amberlite 200 | 9 | 61% | " |
| Lewatit SP 1080 | 6 | 61% | " |
| Lewatit SC 102 | 15 | 61% | " |
| Lewatit SPC 118 | 7 | 64% | " |

Beispiel 9

Eine Lösung aus 50 g Citronellol, 50 ml Wasser und 300 ml Isobutanol wurde 10 Stunden lang bei 80°C mit 100 ml des stark sauren Kationenaustauschers Lewatit SC 102, der zuvor mit dem 5-fachen Volumen eines Isobutanol-Wasser-Gemisches (Mischungsverhältnis 6:1) ausgewaschen worden war, gerührt. Es waren ca. 45% Citronellol umgesetzt und die Gleichgewichtseinstellung erreicht.

Als Nebenprodukt waren ca. 4% 3,7-Dimethyl-7-iso-butoxy-octanol-1 nachzuweisen. Aus dem vom Ionenaustauscher abgetrennten Reaktionsgemisch wurden Hydroxycitronellol in 84 prozentiger Ausbeute, bezogen auf umgesetztes Citronellol, destillativ abgetrennt.

Beispiel 10

Eine Lösung aus 50 g Citronellol, 200 ml Wasser und 600 ml Butanol-(2) wurde 12 Stunden lang bei 80°C mit 100 ml des stark sauren Kationenaustauschers Lewatit SPC 108, der zuvor mit dem 5-fachen Volumen eines Butanol-(2)-Wasser-Gemisches (Mischungsverhältnis 3:2) ausgewaschen worden war, gerührt. Danach waren ca. 47% Citronellol umgesetzt und die Gleichgewichtseinstellung erreicht.

Aus dem vom Ionenaustauscher abgetrennten Reaktionsgemisch wurden destillativ 32 g Hydroxycitronellol erhalten, was einer Ausbeute von 90%, bezogen auf umgesetztes Citronellol, entspricht.

**Patentanspruch**

1. Verfahren zur Herstellung von Hydroxycitronellol (I) durch Anlagerung von Wasser an Citronellol (II), dadurch gekennzeichnet, daß man eine homogene Lösung, enthaltend pro Kilogram II 0,1 bis 10 Liter, vorzugsweise 1 bis 5 Liter Wasser, sowie ein $\alpha$-methylverzweigtes primäres, sekundäres oder tertiäres $C_3$—$C_5$-Alkanol bei 40 bis 120°C mit einem stark sauren Kationenaustauscher behandelt und das Reaktionsgemisch danach wie üblich destillativ auf das gewünschte Verfahrensprodukt aufarbeitet.

**Revendication**

1. Procédé pour la préparation d'hydroxycitronellol (I) avec fixation par addition d'eau sur le citronellol (II), caractérisé en ce qu'on traite par un échangeur de cations fortement acide, à une température de 40 à 120°C, une solution homogène contenant par kilogramme de II, 0,1 à 10 litres, de préférence 1 à 5 litres d'eau, ainsi qu'un alcanol primaire, secondaire ou tertiaire à 3-5 C, $\alpha$-méthyl-ramifié, et en ce qu'on soumet ensuite le mélange réactionnel à un traitement usuel de distillation pour obtenir le produit final voulu.

**Claim**

1. A process for the preparation of hydroxycitronellol (I) by addition reaction of water with citronellol (II), characterized in that a homogeneous solution, containing per kilogram of II, from 0.1 to 10 litres, preferably from 1 to 5 litres, of water as well as an $\alpha$-methyl-branched primary, secondary or tertiary $C_3$—$C_5$-alkanol is treated with a strongly acid cation exchanger at from 40 to 120°C and thereafter the reaction mixture is worked up in the conventional manner by distillation to give the desired product.